# EUROPEAN PATENT APPLICATION

(11) **EP 0 577 274 A2**
(43) Date of publication of application: **05.01.1994**
(21) Application number: 93304457.0
(22) Date of filing: 08.06.1993
(51) Int. Cl.: C12P 17/02, C12N 5/04

(54) **Process for preparing taxane compounds**

(30) Priority: 11.06.1992 JP 177405/92
(71) Applicant: Nippon Oil Co., Ltd., Tokyo 105 (JP)
(72) Inventor: Dicosmo, Frank, Toronto, Ontario (CA); Neto, Arthur g. Fett, Toronto, Ontario (CA)
(74) Representative: Geering, Keith Edwin

(57) **Abstract**

A process for preparing a taxane compound is disclosed. The process comprises providing culture cells originating from a plant belonging to *Taxus* species, culturing the cells in a medium to which an aromatic amino acid or an aromatic carboxylic acid, or both, are added, and collecting a plant component having the taxane structure from the cultured material. The prodcutivity of taxane compounds, which possesses anti-cancer activity, is greatly promoted by the process.

## Description

### BACKGROUND OF THE INVENTION

### Field of the Invention:

The present invention relates to a process for preparing taxane compounds, comprising culturing cells of a plant belonging to *Taxus* species, producing a plant component possessing the taxane structure from the cultured product, and collecting the taxane compounds. In particular, the present invention relates to a process for producing a taxane compound at a high yield from callus or a culture liquid of cells originating from a plant of *Taxus* sp. and collecting the taxane compound.

### Description of the Background Art:

Conventionally, taxol and various derivatives of taxol are known to be present in plants of *Taxus* species. Taxol compounds are also known to exhibit a high anti-cancer activity to various cell lines such as melanoma B16. There are a number of reports concerning clinical test results of the effectiveness of taxol compounds as an anti-cancer component.

A large quantity of taxol compounds is required for the pharmacological tests and a commercial base chemical therapy using the taxol compounds. However, no taxol compound from natural sources has been manufactured in a large scale. Since taxol compounds have very complicated structures, their production by chemical synthesis have not been successful up to the present time. Therefore, various derivatives of taxol compounds have been prepared from materials of natural origin.

Plants of *Taxus* sp. which comprises taxol and various derivatives of taxol are very special plants; their growth is very slow and they are not artificially planted. A bulk of plants of *Taxus* sp. are needed in a large quantity for the production of taxol compounds. In addition, complicated steps are required for the separation and recovery of taxol compounds from such a bulk. Furthermore, there has been a problem that recovered products tend to change very easily.

More recently, a study is reported to be ongoing concerning the production of alkaloid compounds containing the taxane structure by culturing living tissues of a plant of *Taxus* sp. in a specific medium, and by separating and collecting such compounds. This is a new technique as compared with the conventional natural bulk extraction methods in that alkaloid compounds containing the taxane structure are produced by a tissue culture method using living tissues of a plant of *Taxus* sp.

The technology heretofore reported, however, is unsatisfactory due to its low productivity of the alkaloid compounds. The productivity is quite insufficient to satisfy the need in research and development concerning the use and applications of these compounds, such as pharmacological tests, a commercial-base chemical therapy, and the like, all requiring these compounds in a large quantity. Therefore, there are strong needs for the development of a new production technology which is capable of producing these alkaloid compounds having the taxane structure at a high productivity.

In view of this situation, the present inventors have undertaken extensive studies in order to develop a novel, high productivity process for producing taxane compounds, such as taxol compounds, baccatin compounds, and the like, which are highly useful as an anti-cancer component. As a result, the present inventors have found that the productivity of taxane compounds is greatly promoted as compared with conventional tissue culture methods by culturing cells originating from a plant belonging to *Taxus* species in a medium to which a specific component is added. This finding has led to the completion of the present invention.

### SUMMARY OF THE INVENTION

Accordingly, an object of the present invention is to provide a novel process for preparing taxane compounds, such as taxol compounds, baccatin compounds, and the like, which exhibit anti-cancer activity, by the culture of cells from a plant belonging to *Taxus* species at an extremely high productivity.

Another object of the present invention is to provide a novel process for preparing taxane compounds at a productivity extremely higher than conventional processes by the tissue culture of cells from a plant belonging to *Taxus* species.

Still another object of the present invention is to provide a process for preparing taxane compounds, which comprises providing culture cells originating from a plant belonging to *Taxus* species, culturing the cells in a medium to which an aromatic amino acid or an aromatic carboxylic acid, or both, are added, and collecting a plant component having the taxane structure from the cultured material.

Other and further objects, features and advantages of the present invention will appear more fully from the following description.

### BRIEF DESCRIPTION OF THE DRAWINGS

Figure 1 is a graph showing the relationship between the phenylalanine concentration added and the taxol content (%) of the cells, when the cells of *Taxus cuspidata* were cultured in Example 1.

Figure 2 is a similar graph showing the relationship between the phenylalanine concentration added and the baccatin content (%).

### DETAILED DESCRIPTION OF THE INVENTION AND PREFERRED EMBODIMENTS

*Taxus brevifolia, Taxus canadensis, Taxus x media cv. hicksii, Taxus cuspidata,* and the like are given as examples of plants belonging to *Taxus* sp. used as a source of cells to be cultured in the process of the present invention.

Cells from any appropriate parts or tissues of plants belonging to *Taxus* sp., such as roots, leaves, embryos, stems, meristems, and the like, can be used as induction materials of the cells to be cultured by the process of the present invention. They are used in the form of sliced fragments of living tissues from plants belonging to *Taxus* sp. Sprout, young leaves, contents of matured seeds, embryos in the seed contents, red aril, green aril, and the like are used for producing such fragments.

As the cells of *Taxus* sp. plants to be cultured in the process of the present invention, callus derived from the sliced fragments of the plant, liquid cell culture obtained by culturing the callus in a liquid medium, immobilized cells prepared by immobilizing liquid cultured cells on a carrier, or differentiated organs derived from plants of *Taxus* sp., and the like can be used.

For inducing callus, the above plant fragments are sterilized and cultured in a solid medium, such as agar medium, containing a callus-inducing component. The sterilization can be carried out by a surface sterilization treatment using ethanol, a treatment with an aqueous solution of hypochlorous acid, washing with sterilized distilled ion-exchanged water, or the like. The medium containing the callus-inducing component comprises a base medium such as Gamborg B5, Murashige-Skoog, Nitch-Nitch, or the like, which contains inorganic salts and vitamins; as a carbon source, a sugar such as sucrose, glucose, and the like; a plant hormone such as auxins (e.g, 2,4-dichlorophenoxyacetic acid (2,4-D), naphthaleneacetic acid (NAA), indolebutyric acid, indoleacetic acid, etc.), cytokinins (e.g., benzyladenine, kinetin, etc.), and the like; a browning preventive agent; and a gelling agent such as agar, carageenan, and the like. The culture medium generally becomes brown during the course of the cultivation due to colored substances produced by the polymerization of quinone compounds which are oxidation products of phenol compounds. The addition of a browning preventive agent prevents the culture from becoming brown. The concentration of plant hormones also affects the callus induction efficiency, the browning, and the growth of the callus. The amount of plant hormones used can be determined according to the type of the medium and the culture conditions. Taking auxin and cytokinin as examples, a medium comprising 0-4 mg/l of auxin and 0-4 mg/l of cytokinin is preferred. Normally, callus is induced by placing the above-mentioned sliced fragments on a solid medium at a temperature of 15-30°C for a period of 2-8 weeks.

The callus thus induced is sufficiently cultured and grown in the solid medium having the aforementioned composition. A liquid cell culture can be obtained by culturing the callus thus obtained in a liquid medium. The liquid medium is prepared from components mentioned above, except for inclusion of a gelling agent. The liquid culture is normally carried out under the conditions of 15-30°C. The culture is shaken when the culture is carried out in a flask; and an aerobic culture with stirring is applied when the culture is done in a jar fermenter. It is possible to immobilize the cultured cell on water-insoluble carriers. Furthermore, differentiated organs, which may be induced in the course of the induction and growth of callus, can be sufficiently grown in said solid medium or liquid medium under the above-mentioned conditions.

The culture in the solid medium for producing taxane compounds of the present invention can be carried out by inoculating the cells to be cultured in a medium obtained by the addition of aromatic carboxylic acid and/or aromatic amino acid. The culture is carried out by placing the culture materials in a dark place at about 15-30°C, normally, for 20-100 days. The taxane compounds can be directly extracted from the cells cultured in the solid medium and purified; or it is possible to culture such cells in a liquid medium to which aromatic carboxylic acid and/or aromatic amino acid are added to produce the taxane compounds in the culture cell and the culture liquid supernatant, and to extract and purify the taxane compounds. If necessary, it is possible to transfer the liquid culture product to another liquid or solid medium to produce the taxane compounds.

In the present invention, the aromatic amino acid compounds to be added to the solid or liquid medium are the compounds comprising an aromatic functional group (e.g., benzene ring), a nitrogen-containing portion (e.g., amino group, imide group, or their salts), and a carboxylic acid portion (e.g., carboxylic acid, carboxylate, carboxylic acid ester, carboxylic acid anhydride, carboxylic acid amide, carboxylic acid imide, carboxylic acid halide, etc.) in one molecule. Phenylalanine or tyrosine containing phenyl group or monohydoxy phenyl group; 2-amino-2-phenylpropionic acid; 2-amino-2-hydroxyphenylpropionic acid; and the like are given as examples.

The aromatic carboxylic acids used in the present invention are carboxylic acids consisting of an aromatic functional group portion (e.g., benzene ring) and, directly bonded thereto, a carbonyl group portion; and derivatives of such carboxylic acids. Taking compounds having a benzene ring as the aromatic functional group portion as examples, benzoic acid, salts of benzoic acid, esters of benzoic acid, benzoic acid anhydride, benzoic acid amide, benzoyl chloride, and the like are exemplified. For both the aromatic amino acids and aromatic carboxylic acids, salts of the carboxylic acids may be those formed by an alkali metal (e.g., sodium, potassium, etc.) or an alkaline earth metal (e.g., calcium, magnesium, etc.). In addition, these compounds may contain asymmetric carbon atoms and may be optically active compounds. Either racemates or optical isomers may be used for the purpose of the present invention. The productivity of the taxane compounds is greatly promoted by the addition of these compounds to the medium.

Thus, the addition of the above-mentioned compounds to the cultured product is very effective for promoting the productivity of the secondary metabolic products. These compounds are preferably added to the medium in an amount of 0.001 mM to 100 mM. If the amount is less than 0.001 mM, the productivity of the taxane compounds is not promoted; if it is greater than 100 mM, on the other hand, the cell growth is adversely affected. These aromatic amino acids and/or aromatic carboxylic acids may be added at the initial stage of the culture or may be added in the course of the culture. In addition, such aromatic amino acids or aromatic carboxylic acids may be added either individually or after having been dissolved in a suitable surfactant or solvent. Increase in the content of taxol and baccatin III is particularly remarkable when said aromatic amino acid and/or aromatic carboxylic acid, e.g., phenylalanine, is added to the medium to a concentration of 0.01 mM.

The liquid suspension culture can be applicable to the culture of the grown callus in a liquid medium. The addition of glass fiber to the culture liquid for absorbing and immobilizing the cells on the glass fiber significantly promotes the productivity of the taxane compounds. The glass fiber may be of any form. Suitable molded articles can be used.

The culture cell and the culture liquid supernatant thus obtained are dried, and the taxane compounds are extracted therefrom and purified, if required.

Specifically, the taxane compounds produced are extracted with dichloromethane, methanol, hexane, or the like for separation and collection. Any conventional separation and purification methods, such as extraction, column chromatography, and the like, can be applicable to the separation and collection. The extracted liquid after drying may be used as is as the taxane compound, or the taxane compounds may be used after isolation. HPLC analysis and the like are applicable for the analysis of the target taxane compound.

Given as examples of the target taxane compounds are taxol of the following formula (I), cephalomannine of formula (II) (the compound having acetyl group for R in the formula), 10-deacetylcephalomannine of formula (II) (the compound having hydrogen for R in the formula), baccatin III of formula (III) (the compound having acetyl group for R in the formula), and 10-deacetylbaccatin III of formula (III) (the compound having hydrogen for R in the formula).

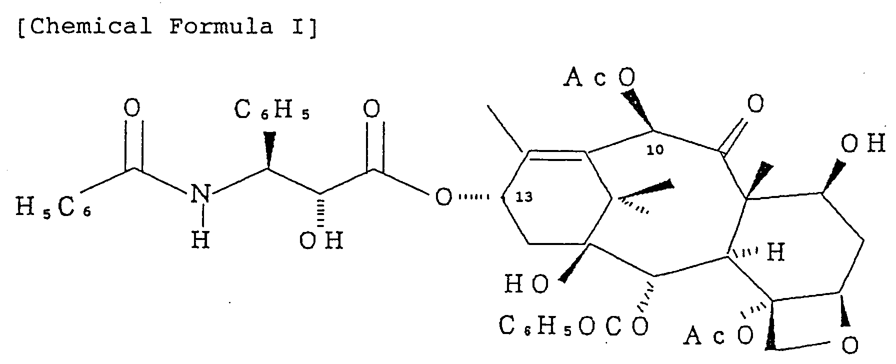

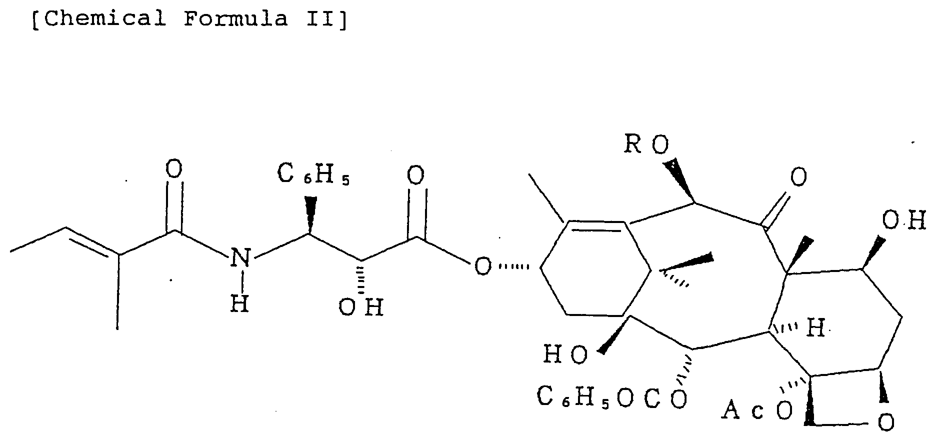

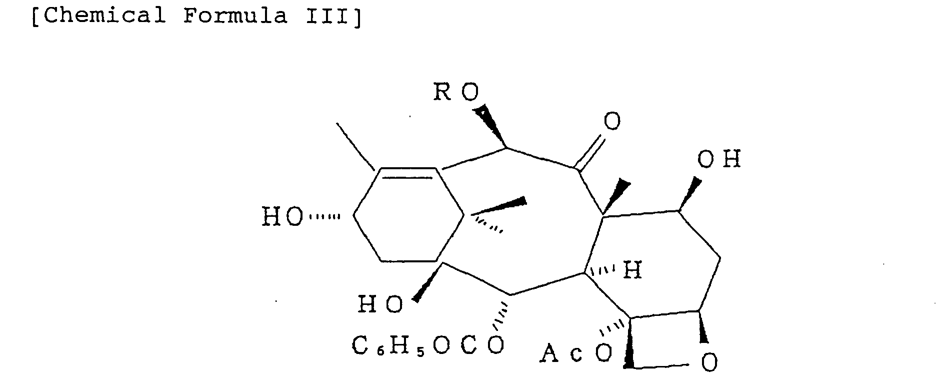

Other features of the invention will become apparent in the following description of the exemplary embodiment which is given for illustration of the invention and is not intended to be limiting thereof.

### EXAMPLE

### Example

Sliced fragments of young stems of *Taxus cuspidata* were washed with distilled water and their surfaces were sterilized with 70% ethanol for 30-60 seconds. The fragments were dipped into 1.5% sodium hypochlorite solution (several drops of Tween were added) for 20-30 minutes and further washed three times with sterilized ion-exchanged water to complete sterilization. The fragments were scattered over a sterilized medium, which was prepared by adding 2% sucrose and 1 mg/l 2,4-dichlorophenoxyacetic acid to Gamborg B5 base medium, on a plastic plate, and the plate was placed in a dark place at 25°C to induce callus. After 15 days, the fragments were transferred to a fresh medium, which was prepared by adding 2% sucrose and 1 mg/l 2,4-dichlorophenoxyacetic acid to Gamborg B5 base medium, to proliferate the callus. Thirty five days thereafter, i.e, 50 days after the start of the culture, media were prepared by adding phenylalanine to said medium, which was prepared by adding 2% sucrose and 1 mg/l 2,4-dichlorophenoxyacetic acid to Gamborg B5 base medium, to concentrations of 0 (control), 0.1, 1.0, 10.0, 100.0 mM, the media were sterilized in an autoclave. The callus was cultured in these media on a plastic plate under dark conditions at 25°C for 25 days.

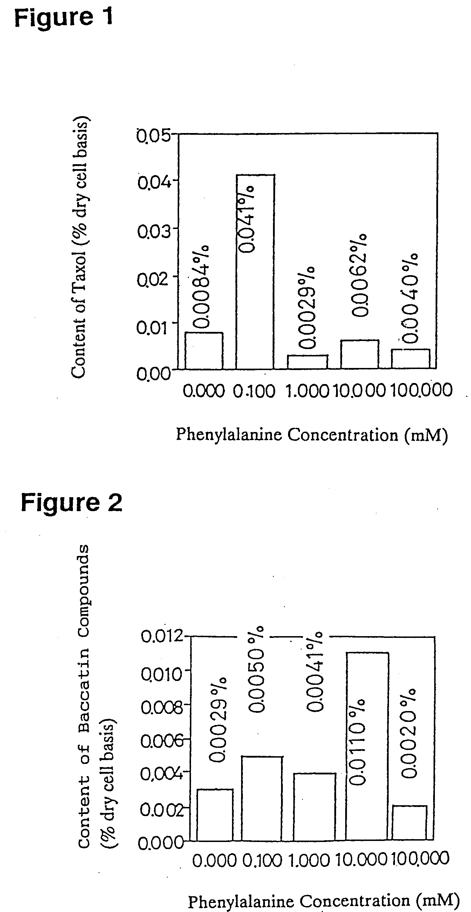

On the 55th day after the initial culture starting date, 0.5 g of fresh callus was sampled, freeze-dried at -20°C, pulverized in 2 ml of hexane, and extracted with hexane at 25°C for 12 hours. The precipitate was separated by centrifuge at 2,500 x g for 20 minutes, extracted with a mixed solvent of methanol and dichloromethane (1:1) under ultrasonic conditions at room temperature for 1 hour. The extract was centrifuged at 2,500 x g for 20 minutes to obtain a supernatant (a crude extract). The crude extract was dried at 25°C and dissolved in 1 ml of dichloromethane. To the solution was added 1 ml of distilled water, followed by mixing for 10 seconds and centrifuge at 2,500 x g for 20 minutes, thus obtaining a dichloromethane layer. This dichloromethane layer was dried at 25°C, dissolved in 500 µl of methanol, and filtered through 0.45 µm filter paper to obtain a sample for HPLC analysis. Taxol compounds and baccatin compounds were detected by the HPLC analysis. Concentrations of these compounds are shown in Figures 1 and 2.

When phenylalanine was added at a concentration of 0.100 mM, the content of the taxol on dry cell basis was 0.041% and that of the baccatin compounds was 0.005%.

According to the process of the present invention, cells originating from plants belonging to *Taxus* species are cultured in a medium containing aromatic amino acid and/or aromatic carboxylic acid, and taxane compounds are collected from the culture product. The process ensures a remarkably promoted productivity in the production of taxane compounds as compared with conventional processes.

Obviously, numerous modifications and variations of the present invention are possible in light of the above teachings. It is therefore to be understood that within the scope of the appended claims, the invention may be practiced other than as specifically described herein.

## Claims

1. A process for preparing a taxane compound comprising,
providing culture cells originating from a plant belonging to *Taxus* species,
culturing the cells in a medium to which an aromatic amino acid or an aromatic carboxylic acid, or both, are added, and
collecting a plant component having a taxane structure from the cultured material.

2. A process according to Claim 1, wherein the amount of said aromatic amino acid or an aromatic carboxylic acid, or both, added is to make a concentration 0.001-100 mM.

3. A process according to Claim 1, wherein said aromatic amino acid is a compound comprising an aromatic functional group portion, a nitrogen-containing portion, and a carboxylic acid portion in one molecule.

4. A process according to Claim 3, wherein said aromatic functional group portion is a benzene ring, said nitrogen-containing portion is selected from the group consisting of amino group, imide group, or salts of amino group or imide group, and said carboxylic acid portion is selected from the group consisting of carboxylic acid, salts of carboxylic acid, esters of carboxylic acid, carboxylic acid anhydride, carboxylic acid amide, carboxylic acid imide, and carboxylic acid halide.

5. A process according to Claim 1, 2, or wherein said aromatic amino acid is selected from the group consisting of phenylalanine, tyrosine, 2-amino-2-phenylpropionic acid, and 2-amino-2-hydroxyphenylpropionic acid.

6. A process according to Claim 1, 2, or 3, wherein said aromatic carboxylic acid is a carboxylic acid consisting of an aromatic functional group portion having a carbonyl group portion directly bonded thereto or a derivative of such a carboxylic acid.

7. A process according to Claim 6, wherein said aromatic carboxylic acid is selected from the group consisting of benzoic acid, esters of benzoic acid, benzoic acid anhydride, benzoic acid amide, and benzoyl chloride.

8. A process according to one of Claims 1-7, wherein the taxane compound is taxol, cephalomannine, 10-deacetylcephalomannine, baccatin III, or 10-deacetylbaccatin III.
